# EUROPÄISCHE PATENTSCHRIFT

(11) **EP 1 309 562 B1**
(45) Veröffentlichungstag und Bekanntmachung des Hinweises auf die Patenterteilung: **07.05.2008**
(21) Anmeldenummer: 01951627.7
(22) Anmeldetag: 22.06.2001
(51) Int. Cl.: C07D 235/02, C07C 227/24, C07C 229/48

(54) **4-ALKOXY-CYCLOHEXAN-1-AMINO-CARBONSÄUREESTER UND VERFAHREN ZU IHRER HERSTELLUNG**
4-ALKOXY CYCLOHEXANE-1 AMINO CARBOXYLIC ACID ESTERS AND METHOD FOR THE PRODUCTION THEREOF
ESTERS D'ACIDE 4-ALCOXYCYCLOHEXAN-1-AMINOCARBOXYLIQUE, ET LEUR PROCEDE DE PRODUCTION

(30) Priorität: 05.07.2000 DE 10032587
(43) Veröffentlichungstag der Anmeldung: 14.05.2003
(73) Patentinhaber: Bayer CropScience AG, 40789 Monheim (DE)
(72) Erfinder: HIMMLER, Thomas, 51519 Odenthal (DE); FISCHER, Reiner, 40789 Monheim (DE); GALLENKAMP, Bernd, 42113 Wuppertal (DE); KNOPS, Hans-Joachim, 40789 Monheim (DE); MULDER, Lubbertus, 58135 Hagen (DE)
(86) Internationale Anmeldenummer: PCT/EP2001/007115
(87) Internationale Veröffentlichungsnummer: WO 2002/002532

(56) Entgegenhaltungen:
- WO-A-97/17092
- WO-A-98/05638
- DATABASE CA [Online] CHEMICAL ABSTRACTS SERVICE, COLUMBUS, OHIO, US; MAKI, YOSHIFUMI ET AL: "1-Amino-4-hydroxycyclohexane-1-carboxylic acid" retrieved from STN Database accession no. 80:3169 XP002180224 -& JP 48 067254 A (MAKI, YOSHIFUMI;MASUGI, TAKASHI) 13. September 1973 (1973-09-13)
- MUNDAY L: "AMINO-ACIDS OF THE CYCLOHEXANE SERIES. PART I" JOURNAL OF THE CHEMICAL SOCIETY, CHEMICAL SOCIETY. LETCHWORTH, GB, Bd. 190, 1961, Seiten 4372-4379, XP000938987 in der Anmeldung erwähnt
- EDWARD J T ET AL: "STEREOCHEMISTRY OF THE BUCHERER-BERGS AND STRECKER REACTIONS OF 4-TERT-BUTYLCYCLOHEXANONE" CANADIAN JOURNAL OF CHEMISTRY, NATIONAL RESEARCH COUNCIL. OTTAWA, CA, Bd. 53, 1975, Seiten 3339-3350, XP000925895 ISSN: 0008-4042 in der Anmeldung erwähnt

## Beschreibung

Die Erfindung betrifft neue 4-Alkoxy-cyclohexan-1-amino-carbonsäureester, Zwischenprodukte und Verfahren zu ihrer Herstellung sowie ihre Verwendung als Zwischenprodukte zur Synthese von insektiziden, akariziden und herbiziden Verbindungen oder pharmazeutischen Wirkstoffen.

Substituierte cyclische Aminocarbonsäuren sind im allgemeinen nach der Bucherer-Bergs-Synthese oder nach der Strecker-Synthese erhältlich und fallen dabei jeweils in unterschiedlichen Isomerenformen an. So erhält man nach den Bedingungen der Bucherer-Bergs-Synthese bei der Herstellung von substituierten cyclischen Aminocarbonsäuren der allgemeinen Formel (I) 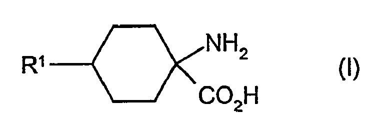 überwiegend das Isomer (I-a), 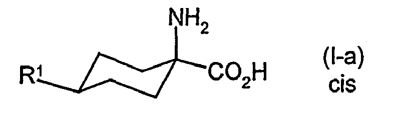 in dem der Rest R¹ und die Aminogruppe cis-ständig angeordnet sind, während nach den Bedingungen der Strecker-Synthese überwiegend das trans-Isomer (I-b) 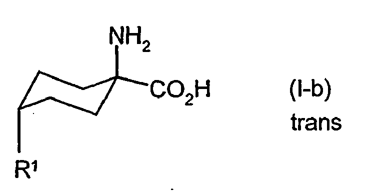 erhalten wird (J. Chem. Soc. 1961, 4372-4379; Chem. Pharm. Bull. 21 (1973) 685-691; Chem. Pharm. Bull. 21 (1973) 2460-2465; Can. J .Chem. 53 (1975) 3339-3350).

Die Bucherer-Bergs-Reaktion wird im allgemeinen derart durchgeführt, dass man ein substituiertes cyclisches Keton der allgemeinen Formel (II) 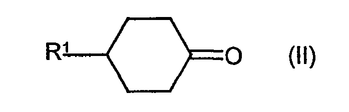 in einem Lösungsmittel oder Lösungsmittelgemisch mit Ammoniumcarbonat und einem Alkalicyanid, im allgemeinen Natrium- oder Kaliumcyanid, umsetzt und das entstandene Hydantoin der allgemeinen Formel (III) 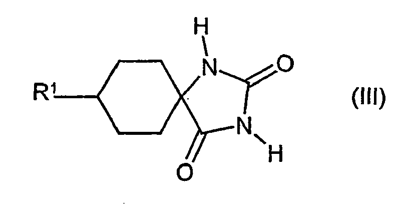 isoliert.

Dabei fallen die Hydantoine der allgemeinen Formel (III) üblicherweise als Gemische der cis-Isomere (III-a) 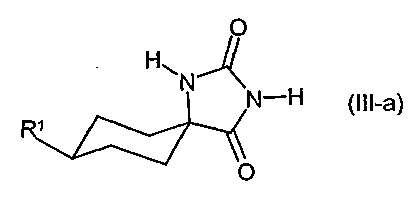 und trans-Isomere (III-b) an

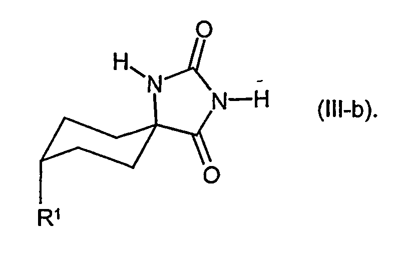

Die Hydantoine der allgemeinen Formel (III) werden anschließend nach bekannten Methoden sauer oder alkalisch zu den substituierten cyclischen Aminocarbonsäuren der allgemeinen Formel (I) verseift.

Die substituierten cyclischen Aminocarbonsäuren der allgemeinen Formel (I) können dann nach bekannten Methoden der organischen Chemie zu den substituierten cyclischen Aminocarbonsäureestern der allgemeinen Formel (IV) 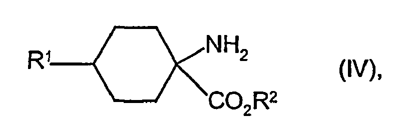 verestert werden.

Verbindungen der Formeln (IV-a) und (IV-b) 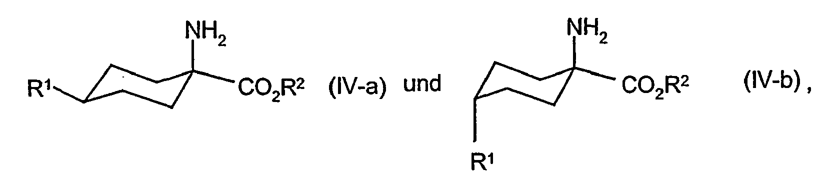 in welcher
- R¹: für OR³,
- R²: für Alkyl und
- R³: für Alkyl steht,
sind teilweise nen.

Für manche Verbindungen (beispielsweise aus EP-A-596298; WO 95/20572, EP-A-668267; WO 95/26954; WO 96/25395; WO 96/35664; WO 97/02243; WO 97/01535; WO 97/36868; WO 98/05638) werden substituierte cyclische Aminocarbonsäureester der allgemeinen Formel (IV) als Vorprodukte benötigt.

Dabei kann es für bestimmte dieser beispielsweise aus EP-A-596298; WO 95/20572; EP-A-668267; WO 95/26954; WO 96/25395; WO 96/35664; WO 97/02243; WO 97/01535; WO 97/36868; WO 98/05638 bekannt gewordenen Verbindungen vorteilhaft sein, sie unter Verwendung von substituierten cyclischen Aminocarbonsäureestem der allgemeinen Formel (IV), in denen das cis-Isomer (IV-a) das alleinige oder zumindest deutlich überwiegende Isomer ist, herzustellen.

Als Lösungsmittel für die Bucherer-Bergs-Reaktion wird im allgemeinen etwa 50%iges wässriges Methanol (J. Org. Chem. 53 (1988) 4069-4074) oder etwa 50%iges wässriges Ethanol (J. Chem. Soc. 1961, 4372-4379; Chem. Pharm. Bull. 21 (1973) 685-691; Chem. Pharm. Bull. 21 (1973) 2460-2465; Can. J. Chem. 53 (1975) 3339-3350; Can. J. Chem. 57 (1979) 1456-1461) verwendet. Auch bei Optimierungen der Bucherer-Bergs-Reaktion wurde wässriges Ethanol als Lösungsmittel verwendet (J. Heterocycl. Chem. 21 (1984) 1527-1531). Ein weiteres für die Bucherer-Bergs-Reaktion bekannt gewordenes Lösungsmittel ist N,N-Dimethylformamid (Helv. Chim. Acta 67 (1984) 1291-1297). Die Verwendung dieser Lösungsmittel bei der Herstellung der Hydantoine der allgemeinen Formel (III) ergibt jedoch unbefriedigende Ausbeuten. Außerdem sind die isolierten Produkte durch anorganische Anteile stark verunreinigt. Nach zusätzlichen Reinigungsoperationen werden schließlich Produkte mit stark wechselnden Zusammensetzungen bzgl. der cis- und trans-Isomeren erhalten, so dass eine konstante Produktqualität nicht gewährleistet werden kann.

Es wurde gefunden, dass man Verbindungen der Formel (III), 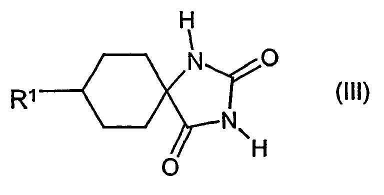 in welcher
- R¹: die oben angegebene Bedeutung hat,
erhält, indem man Verbindungen der Formel (II) 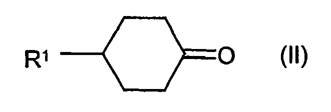 in welcher
- R¹: die oben angegebene Bedeutung hat,
mit Ammoniumcarbonat und Alkalicyaniden oder Trimethylsilylcyanid (TMSCN) in Wasser als Lösungsmittel umsetzt

Überraschenderweise können nach dem erfindungsgemäßen Verfahren die Verbindungen der Formel (III) in hoher Ausbeute und Reinheit sowie einem hohen und reproduzierbaren Anteil des cis-Isomeren (III-a) 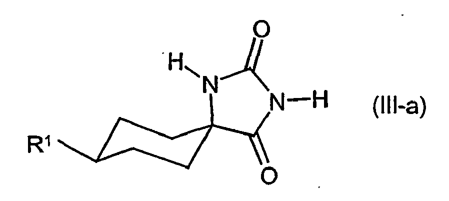 in welcher
- R¹: für OR³ steht,
wobei
- R³: für Alkyl steht,
hergestellt werden.

In den allgemeinen Formeln (II), (III) und (III-a) steht der Rest
- R¹: für OR³,
wobei
- R³: bevorzugt für C₁-C₄-Alkyl steht.

Besonders bevorzugt steht R³ für Methyl, Ethyl, n-Propyl, n-Butyl oder i-Butyl.

Ganz besonders bevorzugt steht R³ für Methyl.

Hervorgehoben ist die Verbindung der Formel (III-a), in welcher R³ für Methyl steht.

Die Verbindungen der Formel (III) und die Isomere der Formel (III-a) und (III-b) sind neu und Gegenstand dieser Erfindung.

In der allgemeinen Formel (III-b) hat die Variable R¹ die oben angegebene Bedeutung.

Verbindungen der Formel (III) lassen sich nach bekannten Methoden zu den Verbindungen der Formel (I), 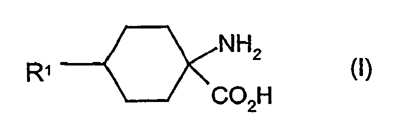 in welcher
- R¹: die oben angegebene Bedeutung hat,
hydrolysieren und anschließend nach bekannten Methoden zu Verbindungen der Formel (IV) verestern.

Als Alkalicyanide können zur Herstellung der Verbindungen der Formel (III) bevorzugt Lithiumcyanid, Natriumcyanid oder Kaliumcyanid eingesetzt werden, besonders bevorzugt sind Natrium- und Kaliumcyanid.

Die Menge an Alkalicyanid oder TMSCN bezogen auf Keton liegt zwischen 0,9 und 3 Mol pro Mol Keton. Bevorzugt werden Mengen zwischen 1 und 2,5 Mol pro Mol Keton eingesetzt; besonders bevorzugt sind Mengen zwischen 1,1 und 2 Mol Alkalicyanid pro Mol Keton.

Die Menge an Ammoniumcarbonat beträgt zwischen 0,5 und 7 Mol Ammoniumcarbonat pro Mol Keton. Bevorzugt werden Mengen zwischen 0,8 und 5 Mol pro Mol Keton eingesetzt; besonders bevorzugt sind Mengen zwischen 1 und 5 Mol Ammoniumcarbonat pro Mol Keton.

Die Reaktionstemperatur des erfindungsgemäßen Verfahrens liegt zwischen 20 und 100°C; bevorzugt ist ein Temperaturbereich von 30 bis 70°C.

Die Reaktion kann auch bei erhöhtem oder vermindertem Druck durchgeführt werden.

Die Isolierung des Reaktionsproduktes erfolgt in einfacher Weise durch Filtration des Reaktionsgemisches und Trocknen des Filterrückstandes. Die Filtration erfolgt bei einer Temperatur zwischen 0 und 40°C, bevorzugt bei einer Temperatur zwischen 15 und 30°C.

Auf diese Weise werden die gewünschten Hydantoine der Formel (III) in hoher Ausbeute und Reinheit mit einem reproduzierbaren Isomerenverhältnis erhalten.

Das erfindungsgemäße Verfahren kann beispielhaft durch folgendes Schema veranschaulicht werden:

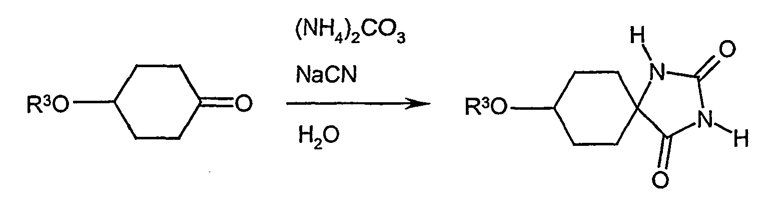

Ebenfalls Gegenstand dieser Erfindung ist ein Verfahren zur Herstellung der Verbindung der Formel (III-a), 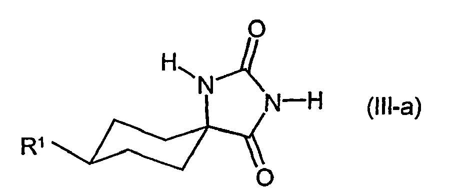 in welcher
- R¹: die oben angegebenen Bedeutungen hat,
dadurch gekennzeichnet, dass man Verbindungen der Formel (II), 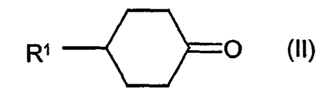 in welcher
- R¹: die oben angegebene Bedeutung hat,
mit einem Alkalicyanid und Ammoniumcarbonat in Wasser umsetzt.

Besonders bevorzugt ist ein Verfahren zur Herstellung der Verbindung der Formel (III-a), in welcher
- R¹: für OR³ steht,
wobei
- R³: für Methyl steht,
dadurch gekennzeichnet, dass man 4-Methoxycyclohexanon mit einem Alkalicyanid und Ammoniumcarbonat in Wasser umsetzt.

Als Alkalicyanide können Lithiumcyanid, Natriumcyanid oder Kaliumcyanid eingesetzt werden; bevorzugt sind Natrium- und Kaliumcyanid. Besonders bevorzugt ist Natriumcyanid.

Die Menge an Alkalicyanid bezogen auf die Verbindung der Formel (II) liegt zwischen 0,9 und 3 Mol pro Mol der Verbindung der Formel (II). Bevorzugt werden Mengen zwischen 0,9 und 2,5 Mol pro Mol der Verbindung der Formel (II) eingesetzt; besonders bevorzugt sind Mengen zwischen 1 und 2 Mol Alkalicyanid pro Mol der Verbindung der Formel (II).

Gleichzeitig beträgt die Menge an Ammoniumcarbonat zwischen 0,8 und 2 Mol Ammoniumcarbonat pro Mol der Verbindung der Formel (II). Bevorzugt werden Mengen zwischen 1 und 1,8 Mol pro Mol der Verbindung der Formel (II) eingesetzt.

Die Menge an Wasser als Lösungsmittel liegt zwischen 500 und 3000 ml Wasser pro Mol der Verbindung der Formel (II); bevorzugt ist eine Menge an Wasser von 1000 bis 2500 ml pro Mol der Verbindung der Formel (II).

Die Reaktionstemperatur des erfindungsgemäßen Verfahrens liegt zwischen 20 und 100°C; bevorzugt ist ein Temperaturbereich von 30 bis 70°C.

Die Isolierung des Reaktionsproduktes erfolgt in einfacher Weise durch Filtration des Reaktionsgemisches und Trocknen des Filterrückstandes. Die Filtration erfolgt bei einer Temperatur zwischen 0 und 40°C, bevorzugt bei einer Temperatur zwischen 0 und 20°C.

Ebenfalls Gegenstand dieser Erfindung ist ein Verfahren zur Isolierung der Verbindung der Formel (III-a), 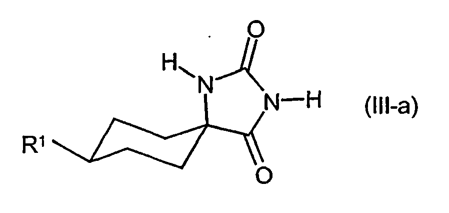 in welcher
- R¹: die oben angegebenen Bedeutungen hat,
dadurch gekennzeichnet, dass man Verbindungen der Formel (III) (cis/trans-Gemische (III-a)/(III-b)) mit wässrigem Ammoniak behandelt und den ungelöst verbleibenden Feststoff in bekannter Weise isoliert.

Die Menge an Ammoniak bezogen auf das im Gemisch vorliegende trans-Isomer der Formel (III-b) liegt zwischen 1 und 30 Mol pro Mol des trans-Isomers der Formel (M-b). Bevorzugt werden Mengen zwischen 4 und 20 Mol pro Mol des trans-Isomers der Formel (III-b) eingesetzt; besonders bevorzugt sind Mengen zwischen 6 und 15 Mol Ammoniak pro Mol des trans-Isomers der Formel (III-b).

Die Menge an Wasser als Lösungsmittel liegt zwischen 500 und 3000 ml Wasser pro Mol der Verbindung der Formel (III); bevorzugt ist eine Menge an Wasser von 1000 bis 2500 ml pro Mol der Verbindung der Formel (III).

Die Temperatur des erfindungsgemäßen Verfahrens liegt zwischen 0 und 100°C; bevorzugt ist ein Temperaturbereich von 10 bis 60°C.

Die Hydantoine der allgemeinen Formel (III) lassen sich nach bekannten Methoden zu den Aminosäuren der allgemeinen Formel (I) hydrolysieren und anschließend nach bekannten Methoden zu Verbindungen der Formel (IV) verestern.

Ebenfalls Gegenstand der vorliegenden Erfindung sind substituierte cyclische Aminocarbonsäuren der allgemeinen Formel (I) 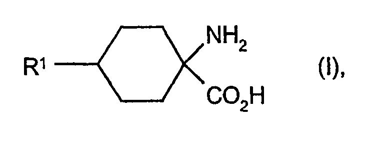 in der
- R¹: für OR³ steht,
wobei
- R³: für Alkyl, bevorzugt C₁-C₄-Alkyl steht.

Die substituierten cyclischen Aminocarbonsäuren der allgemeinen Formel (I) können sowohl als Gemische der cis-Isomeren (I-a) und trans-Isomeren (I-b) vorliegen oder als reine Isomere.

Die Verbindungen der Formel (I) sind neu und Gegenstand dieser Erfindung.

Besonders bevorzugt sind Verbindungen der allgemeinen Formel (I), in der
- R¹: für OR³ steht,
wobei
- R³: für Methyl oder Ethyl steht.

Ganz besonders bevorzugt sind Verbindungen der allgemeinen Formel (I-a), in der
- R¹: für OR³ steht,
wobei
- R³: für Methyl oder Ethyl steht.

Substituierte cyclische Aminocarbonsäuren der Formel (I) bzw. Aminocarbonsäureester der Formel (IV) sind Zwischenprodukte bei der Herstellung anderer Verbindungen, die beispielsweise als Wirkstoffe im Pflanzenschutz oder als pharmazeutische Wirkstoffe Anwendung finden.

So ist z.B. aus der EP-A-596 298, WO 95/20572, EP-A-668 267, WO 95/26954, WO 96/25395, WO 96/35664, WO 97/02243, WO 97/01535, WO 97/36868, WO 98/05638 bekannt geworden, dass substituierte cyclische Aminocarbonsäuren zur Herstellung von substituierten Phenylketoenolen benötigt werden, die als Schädlingsbekämpfungsmittel und Herbizide Anwendung finden können.

Die folgenden Beispiele erläutern den Gegenstand der Erfindung ohne sie in irgendeiner Weise einzuschränken.

### Herstellunssbeispiele

### Vergleichsbeispiel 1

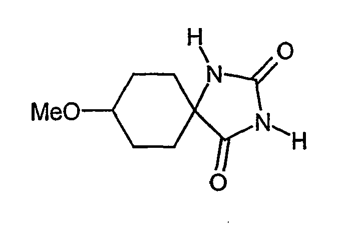

In 110 ml Wasser werden 26,9 g [280 mmol] Ammoniumcarbonat und 5,88 g [120 mmol] Natriumcyanid vorgelegt. Bei Raumtemperatur beginnend wird eine Lösung von 7,7 g [60 mmol] 4-Methoxy-cyclohexanon in 110 ml Ethanol zugetropft. Das Reaktionsgemisch wird 16 Stunden bei 55 bis 60°C gerührt und anschließend vollständig eingeengt (das cis/trans-Verhältnis beträgt nach HPLC 66:34). Das Rohprodukt wird mit 100 ml 50 %igem wässrigen Ethanol 1 Stunde verrührt, auf 0 bis 5°C abgekühlt, 1 Stunde bei 0 bis 5°C gerührt und filtriert. Nach Trocknen des Filterrückstandes erhält man 12,07 g Feststoff mit einem Produktgehalt von 57,8 % (HPLC gegen Standard), womit sich eine Ausbeute von 58,7 % der Theorie ergibt; das cis/trans-Verhältnis beträgt 91:9. Die Elementaranalyse ergibt einen Natriumgehalt von 16 %.

### Vergleichsbeispiel 2

Es wurde vorgegangen wie in Vergleichsbeispiel 1. Nach der Aufarbeitung wurde ein Produkt mit einem cis/trans-Verhältnis von 80:20 erhalten.

### Beispiel 1

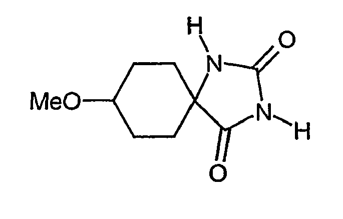

In 560 ml Wasser werden 134,6 g [1,4 mol] Ammoniumcarbonat und 29,4 g [0,6 mol] Natriumcyanid vorgelegt. Bei Raumtemperatur beginnend werden 38,5 g [0,3 mol] 4-Methoxy-cyclohexanon zugetropft. Das Reaktionsgemisch wird 16 Stunden bei 55 bis 60°C gerührt, auf 0 bis 5°C abgekühlt und 2 Stunden bei dieser Temperatur gerührt. Der Feststoff wird abgesaugt und getrocknet. Man erhält 57,88 g Feststoff mit einem Produktgehalt von 93,4% (HPLC gegen Standard), womit sich eine Ausbeute von 90,9% der Theorie ergibt; das cis/trans-Verhältnis beträgt 71:29. Die Elementaranalyse ergibt einen Natriumgehalt von 1,2 %.

### Beispiel 2

In 560 ml Wasser werden 134,6 g [1,4 mol] Ammoniumcarbonat und 22,05 g [0,45 mol] Natriumcyanid vorgelegt. Bei Raumtemperatur beginnend werden 38,5 g [0,3 mol] 4-Methoxy-cyclohexanon zugetropft. Das Reaktionsgemisch wird 4 Stunden bei 55 bis 60°C gerührt, auf 0 bis 5°C abgekühlt und 2 Stunden bei dieser Temperatur gerührt. Der Feststoff wird abgesaugt und getrocknet. Man erhält 57,64 g Feststoff mit einem Produktgehalt von 93,7 % (HPLC gegen Standard), womit sich eine Ausbeute von 90,8 % der Theorie ergibt; das cis/trans-Verhältnis beträgt 72:28. Die Elementaranalyse ergibt einen Natriumgehalt von 1,3 %.

### Beispiel 3

In 560 ml Wasser werden 134,6 g [1,4 mol] Ammoniumcarbonat und 16,17 g [0,33 mol] Natriumcyanid vorgelegt. Bei Raumtemperatur beginnend werden 38,5 g [0,3 mol] 4-Methoxy-cyclohexanon zugetropft. Das Reaktionsgemisch wird 4 Stunden bei 55 bis 60°C gerührt, auf 0 bis 5°C abgekühlt und 2 Stunden bei dieser Temperatur gerührt. Der Feststoff wird abgesaugt und getrocknet. Man erhält 61,02 g Feststoff mit einem Produktgehalt von 94,1% (HPLC gegen Standard), womit sich eine Ausbeute von 96,5% der Theorie ergibt; das cis/trans-Verhältnis beträgt 71:29.

### Beispiel 4

Es wird vorgegangen wie in Beispiel 3. Man erhält 59,54 g Feststoff mit einem Produktgehalt von 93,6 % (HPLC gegen Standard), womit sich eine Ausbeute von 93,7 % der Theorie ergibt; das cis/trans-Verhältnis beträgt 71:29.

### Beispiel 5

In 560 ml Wasser werden 134,6 g [1,4 mol] Ammoniumcarbonat und 16,17 g [0,33 mol] Natriumcyanid vorgelegt. Bei Raumtemperatur beginnend werden 38,5 g [0,3 mol] 4-Methoxy-cyclohexanon zugetropft. Das Reaktionsgemisch wird 4 Stunden bei 55 bis 60°C und anschließend über Nacht bei Raumtemperatur gerührt. Der Feststoff wird bei Raumtemperatur abgesaugt und getrocknet. Man erhält 58,5 g Feststoff mit einem Produktgehalt von 95,4 % (HPLC gegen Standard), womit sich eine Ausbeute von 93,9% der Theorie ergibt; das cis/trans-Verhältnis beträgt 71:29.

### Beispiel 6

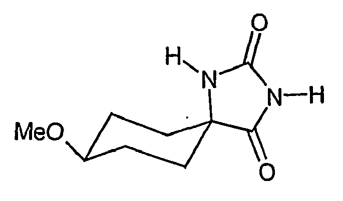

In 560 ml Wasser werden 43,2 g [0,45 mol] Ammoniumcarbonat und 29,4 g [0,6 mol] Natriumcyanid vorgelegt. Bei Raumtemperatur beginnend werden 38,5 g [0,3 mol] 4-Methoxy-cyclohexanon zugetropft. Das Reaktionsgemisch wird 4 Stunden bei 55 bis 60°C gerührt, auf 0 bis 5°C abgekühlt und 2 Stunden bei dieser Temperatur gerührt. Der Feststoff wird abgesaugt und getrocknet. Man erhält 26,4 g Feststoff, womit sich eine Ausbeute von 44,4 % der Theorie ergibt; das cis/trans-Verhältnis ist > 99,7 : 0,3.
Schmelzpunkt: 267-268°C (Sublimation).
¹H-NMR (400 MHz, d-DMSO): δ = 1,38 - 1,48 (m; 2H), 1,57 - 1,68 (m; 4H), 1,91 - 1,95 (m; 2H), 3,14 - 3,17 (m; 1H), 3,23 (s; 3H), 8,37 (s; 1H) ppm.

### Beispiel 7

In 560 ml Wasser werden 34,6 g [0,36 mol] Ammoniumcarbonat und 29,4 g [0,6 mol] Natriumcyanid vorgelegt. Bei Raumtemperatur beginnend werden 38,5 g [0,3 mol] 4-Methoxy-cyclohexanon zugetropft. Das Reaktionsgemisch wird 4 Stunden bei 55 bis 60°C gerührt, auf 0 bis 5°C abgekühlt und 2 Stunden bei dieser Temperatur gerührt. Der Feststoff wird abgesaugt und getrocknet. Man erhält 18,8 g Feststoff, womit sich eine Ausbeute von 31,6 % der Theorie ergibt; das cis/trans-Verhältnis ist 99,4 : 0,6.

### Beispiel 8

In 560 ml Wasser werden 28,8 g [0,3 mol] Ammoniumcarbonat und 16,2 g [0,33 mol] Natriumcyanid vorgelegt. Bei Raumtemperatur beginnend werden 38,5 g [0,3 mol] 4-Methoxy-cyclohexanon zugetropft. Das Reaktionsgemisch wird 4 Stunden bei 55 bis 60°C gerührt, auf 0 bis 5°C abgekühlt und 2 Stunden bei dieser Temperatur gerührt. Der Feststoff wird abgesaugt und getrocknet. Man erhält 15,5 g Feststoff, womit sich eine Ausbeute von 26,1% der Theorie ergibt; das cis/trans-Verhältnis ist 99,2 : 0,8.

### Beispiel 9

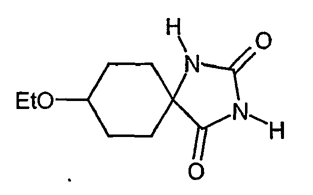

In 56 ml Wasser werden 13,5 g [140 mmol] Ammoniumcarbonat und 1,62 g [33 mmol] Natriumcyanid vorgelegt. Bei Raumtemperatur beginnend werden 4,3 g [30 mmol] 4-Ethoxy-cyclohexanon zugetropft. Das Reaktionsgemisch wird 4 Stunden bei 55 bis 60°C gerührt, auf 0 bis 5°C abgekühlt und 2 Stunden bei dieser Temperatur gerührt. Der Feststoff wird abgesaugt und getrocknet. Man erhält 5,55 g Feststoff (78,8 % der Theorie); das cis/trans-Verhältnis beträgt 72:28.
¹H-NMR (400 MHz, d-DMSO): δ = 1,09 (t; 3H, cis), 1,12 (t; 3H, trans), 1,3 - 1,48 (m; 2H, cis + trans), 1,57 - 1,64 (m; 4H, cis + trans), 1,77 - 1,95 (m; 2H, cis + trans), 3,25 - 3,3 (m; 1H, cis + trans), 3,40 (q; 2H, trans), 3,45 (q; 2H, cis), 8,40 (s, br; 1H, cis + trans) ppm.

Als weitere Beispiele der Formel (III) 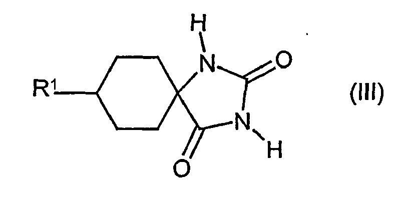 seien genannt:

| | | | |
|---|---|---|---|
| **Beispiel 10:** | R¹=O-ⁿC₃H₇ | Fp. >·250°C | cis/trans = 87/13 |
| | | | |
| **Beispiel 11:** | R¹=O-ⁿC₄H₉ | Fp. > 250°C | cis/trans = 85/15 |
| | | | |
| **Beispiel 12:** | R¹=O-ⁱC₄H₉ | Fp. > 250°C | cis/trans = 51/49 |

### Beispiel 13

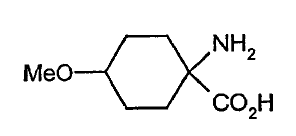

19,8 g [0,1 mol] 4-Methoxycyclohexan-1-spiro-5'-hydantoin (cis/trans-Verhältnis 71:29), 4 g [0,1 mol] Natriumhydroxid und 400 ml Wasser werden in einem Autoklaven 24 Stunden auf 160°C erhitzt. Das Reaktionsgemisch wird unter Eiskühlung mit Salzsäure auf pH 3 gestellt und unter vermindertem Druck weitgehend eingeengt. Das restliche Wasser wird durch Azeotropdestillation mit Toluol entfernt. Man erhält 29,6 g Feststoff.

Lt. GC/MS (nach Silylierung) liegen noch 3,7% Ausgangsmaterial und 89,3% 4-Methoxycyclohexan-1-amino-carbonsäure vor; das cis/trans-Verhältnis beträgt 70:30.

GC/MS(sil.): m/e = 302 (Produkt (zweimal silyliert) - 15), 200 (Basepeak, Produkt (zweimal silyliert) - CO₂SiMe₃), 168 (200 - MeOH).

### Beispiel 14

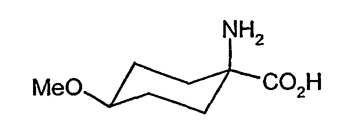

7,9 g [40 mmol] cis-4-Methoxycyclohexan-1-spiro-5'-hydantoin, 160 ml Wasser und 1,6 g [40 mmol] Natriumhydroxid werden in einem Autoklaven 24 Stunden auf 160°C erhitzt. Das Reaktionsgemisch wird unter Eiskühlung mit Salzsäure auf pH 3 gestellt und unter vermindertem Druck weitgehend eingeengt. Das restliche Wasser wird durch Azeotropdestillation mit Toluol entfernt. Man erhält 11,2 g Feststoff. Fp. >400°C
¹H-NMR (400 MHz, d₆-DMSO): δ = 3,17 (m, 1H, CHOCH₃), 3,22 (s, 3H, OCH₃) ppm.

### Beispiel 15

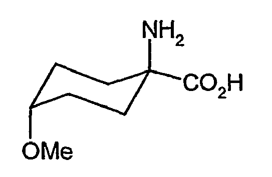

1 g [5 mmol] trans-4-Methoxycyclohexan-1-spiro-5'-hydantoin, 20 ml Wasser und 0,2 g [5 mmol] Natriumhydroxid werden in einem Autoklaven 24 Stunden auf 160°C erhitzt. Das Reaktionsgemisch wird unter Eiskühlung mit Salzsäure auf pH 3 gestellt und unter vermindertem Druck weitgehend eingeengt. Das restliche Wasser wird durch Azeotropdestillation mit Toluol entfernt.
Man erhält 0,8 g Feststoff.

### Beispiel 16

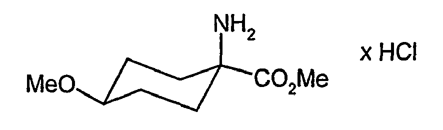

6,9 g [40 mmol] cis-4-Methoxycyclohexan-1-aminocarbonsäure werden in 50 ml wasserfreien Methanol suspendiert. Das Gemisch wird kurz bis zum Rückfluss erhitzt und dann auf 0°C abgekühlt. Bei 0 bis 5°C werden 6,9 g [58 mmol] Thionylchlorid zugetropft. Man rührt eine halbe Stunde bei 0 bis 5°C, lässt dann auf Raumtemperatur kommen, erwärmt auf 40°C und rührt über Nacht bei 40°C. Das Reaktionsgemisch wird filtriert, der Filterrückstand mit 20 ml Methanol gewaschen und das Filtrat eingeengt. Der Rückstand wird mit 50 ml Methyl-tertiärbutyl-ether verrührt, abgesaugt und der Rückstand getrocknet. Man erhält 5,6 g cis-4-Methoxycyclohexan-1-aminocarbonsäuremethylester-Hydrochlorid (63 % der Theorie). Fp. 298°C
¹H-NMR (400 MHz, d-DMSO): δ = 1,64 - 1,80 (m; 4H), 1,88 - 1,96 (m; 4H), 3,23 (s; 3H), 3,29 - 3,32 (m; 1H), 3,76 (s; 3H), 8,67 (s, br; 3H) ppm.

### Beispiel 17

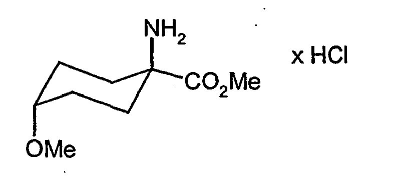

In gleicher Weise wie in Beispiel 12 beschrieben wird trans-4-Methoxycyclohexan-1-aminocarbonsäuremethylester-Hydrochlorid hergestellt.
Fp. 173°C
¹H-NMR (400 MHz, d₆-DMSO): δ =185-2,37 (4 m, 8H, CH₂), 3,32 (s, 3H, CHOCH₃), 3,50 ("d", 1H, CHOCH₃), 3,82 (s, 3H, OCH₃), 8,94 (br, 3H, ^{⊕}NH₃) ppm.

In Analogie zu Beispiel 15 erhält man folgende Aminosäureester der Formel (IV)

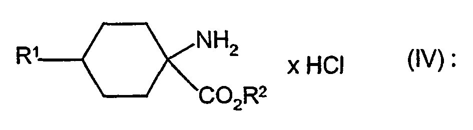

| | | | |
|---|---|---|---|
| **Bespiel 18:** | R¹ = O-C₂H₅ | R² = Me | Fp. > 220°C |
| | | | |
| **Beispiel 19:** | R¹ = O-ⁿC₃H₇ | R² = Me | Fp. > 220°C |
| | | | |
| **Beispiel 20:** | R¹ = O-ⁿC₄H₉ | R² = Me | Fp. 183°C |
| | | | |
| **Beispiel 21:** | R¹ = O-ⁱC₄H₉ | R² = Me | Fp. 179°C |
| | | | |
| **Beispiel 22:** | R¹ = OMe | R² = Et | MS(silyl.):m/e = 273 (M⁺) |
| | | | |
| **Beispiel 23:** | R¹ = OMe | R² = ⁿBu | ¹H-NMR |
| | ¹H-NMR (400 MHz, d-DMSO): δ = 0,88 - 0,92 (t; 3 H), 1,32 - 1,41 (m; 2 H), 1,57 - 1,68 (m; 2 H), 1,69 - 2,1 (m; 10 H), 3,23 (s; 3 H), 3,27 - 3,31 (m; 1H), 4,14 - 4,18 (m; 2 H), 8,77 (s, br; 3 H) ppm. | | |

### Beispiel 24

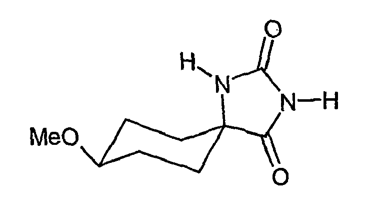

In 86 ml Wasser und 9,8 g 26 %igem Ammoniak werden 10,2 g Verbindung der Formel (III) mit R¹ = OR³, wobei R³ für Methyl steht (8-Methoxy-1,3-diazaspiro[4.5]decan-2,4-dion; 97 %ig, cis/trans-Verhältnis = 75 : 25) 4 Stunden bei 55°C verrührt. Das Gemisch wird auf 0 bis 5°C abgekühlt und 2 Stunden bei dieser Temperatur gerührt. Der Feststoff wird abgesaugt und getrocknet. Man erhält 5,37 g Feststoff; das cis/trans-Verhältnis beträgt 98,3 : I,7.

### Beispiel 25

Man geht vor wie im Beispiel 24, mit dem Unterschied, daß 4 Stunden bei Raumtemperatur gerührt wird. Man erhält 5,03 g Feststoff mit einem cis/trans-Verhältnis von 97,7 : 2,3.

### Beispiel 26

In 86 ml Wasser und 6,5 g 26 %igem Ammoniak werden 10,2 g Verbindung der Formel (III) mit R¹ = OR³, wobei R³ für Methyl steht (8-Methoxy-1,3-diazaspiro[4.5]decan-2,4-dion; 97%ig, cis/trans-Verhältnis = 75 : 25) 4 Stunden bei 55°C verrührt. Das Gemisch wird auf 0 bis 5°C abgekühlt und 2 Stunden bei dieser Temperatur gerührt. Der Feststoff wird abgesaugt und getrocknet. Man erhält 5,73 g Feststoff; das cis/trans-Verhältnis beträgt 97,3 : 2,7.

### Beispiel 27

In 17 ml Wasser und 0,69 g 26 %igem Ammoniak werden 10,4 g Verbindung der Formel (III) mit R¹ = OR³, wobei R³ für Methyl steht (8-Methoxy-1,3-diazaspiro[4.5]decan-2,4-dion; 95,3 %ig, cis/trans-Verhältnis = 98,2 : 1,8) 4 Stunden bei 55°C verrührt. Das Gemisch wird auf 0 bis 5°C abgekühlt und 2 Stunden bei dieser Temperatur gerührt. Der Feststoff wird abgesaugt und getrocknet. Man erhält 9,58 g Feststoff; das cis/trans-Verhältnis ist > 99,7 : 0,3.

## Patentansprüche

1. Verbindungen der Formel (III) 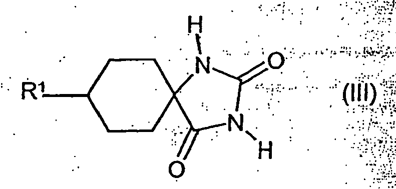 in welcher
R¹ für OR³ steht und R³ für Alkyl steht.

2. Verbindung der Formel (III) gemäß Anspruch 1,
in welcher
R³ für C₁-C₄-Alkyl steht.

3. Verbindung der Formel (III) gemäß Anspruch 1,
in welcher
R³ für Methyl, Ethyl, n-Propyl, n-Butyl oder i-Butyl steht.

4. Verwindungen der Formel (III-a) gemäß Anspruch 1 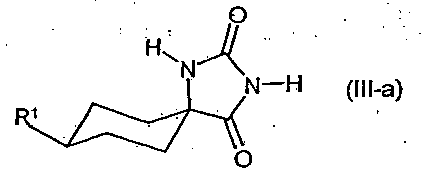 in welcher
R¹ die oben angegebene Bedeutung hat.

5. Verbindungen der Formel (III-b) gemäß Anspruch 1 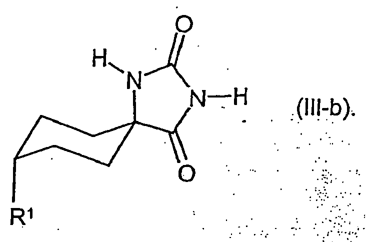 in welcher
R¹ die oben angegebene Bedeutung hat.

6. Verfahren zur Herstellung von Verbindungen der Formel (III), 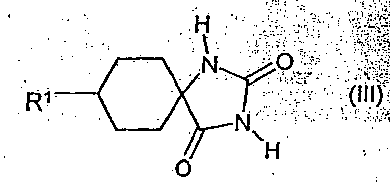 in welcher
R¹ die oben angegebene Bedeutung hat,
**dadurch gekennzeichnet, dass** man Verbindungen der Formel (II) 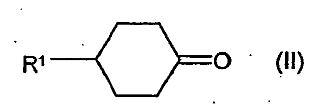 in welcher
R¹ die oben angegebene Bedeutung hat,
mit Ammoniumcarbonat und Alkalicyaniden oder Trimethylsilycyanid (TMSCN) in Wasser als Lösungsmittel umsetzt.

7. Verfahren zur Herstellung der Verbindungen der Formel (III-a) gemäß Anspruch 6 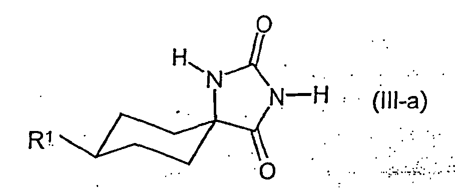 in welcher
R¹ die oben angegebenen Bedeutungen hat,
**dadurch gekennzeichnet, dass** man Verbindungen der Formel (II) 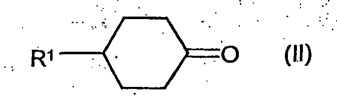 in welcher
R¹ die oben angegebene Bedeutung hat,
mit einem Alkalicyanid und Ammoniumcarbonat in Wasser umsetzt.

8. Verfahren zur Isolierung von Verbindungen der Formel (III-a) 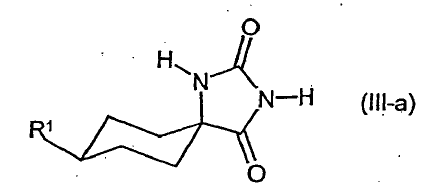 in welcher
R¹ die oben angegebene Bedeutung hat,
**dadurch gekennzeichnet, dass** man Verbindungen der Formel (III) mit wässrigem Ammoniak behandelt.

9. Verbindungen der allgemeinen Formel (I) 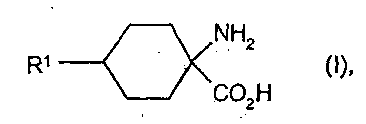 in der
R¹ für OR³ steht,
wobei
R³ für Alkyl steht.

10. Verbindungen der Formel (I) gemäß Anspruch 9,
in welcher
R¹ für OR³ steht,
wobei
R³ für C₁-C₄-Alkyl steht.

11. Verbindungen der Formel (I) gemäß Anspruch 9,
in welcher
R¹ für OR³ steht,
wobei
R³ für Methyl oder Ethyl steht.

12. Verbindungen der Formel (Ia) 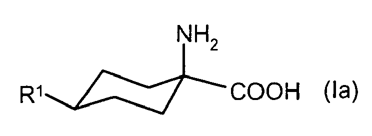 in welcher
R¹ die oben angegebenen Bedeutungen haben.

13. Verbindungen der Formel (Ib) 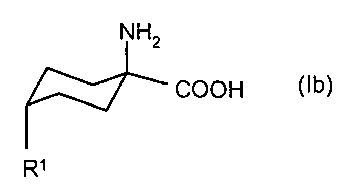 in welcher
R¹ die oben angegebenen Bedeutungen haben.

## Claims

1. Compounds of the formula (III) 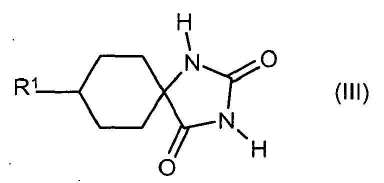 in which
R¹ represents OR³ and R³ represents alkyl.

2. Compounds of the formula (III) according to Claim 1, in which
R³ represents C₁-C₄-alkyl.

3. Compounds of the formula (III) according to Claim 1, in which
R³ represents methyl, ethyl, n-propyl, n-butyl or i-butyl.

4. Compounds of the formula (III-a) according to Claim 1 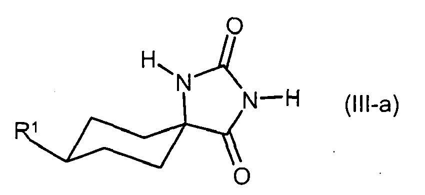 in which
R¹ is as defined above.

5. Compounds of the formula (III-b) according to Claim 1 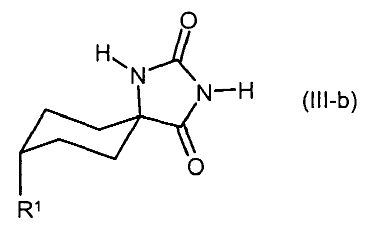 in which
R¹ is as defined above.

6. Process for preparing compounds of the formula (III), 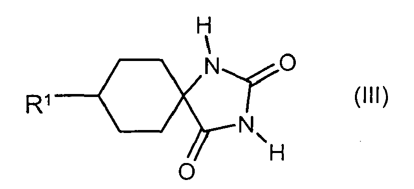 in which
R¹ is as defined above,
**characterized in that** compounds of the formula (II) 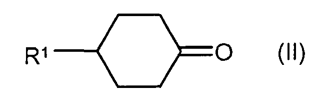 in which
R¹ is as defined above
are reacted with ammonium carbonate and alkali metal cyanides or trimethylsilyl cyanide (TMSCN) in the solvent water.

7. Process for preparing compounds of the formula (III-a) according to Claim 6 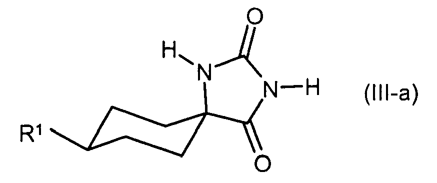 in which
R¹ is as defined above,
**characterized in that** compounds of the formula (II) 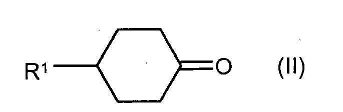 in which
R¹ is as defined above
are reacted with an alkali metal cyanide and ammonium carbonate in water.

8. Process for isolating compounds of the formula (III-a) 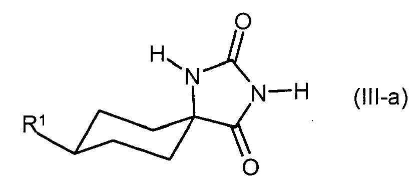 in which
R¹ is as defined above,
**characterized in that** compounds of the formula (III) are treated with aqueous ammonia.

9. Compounds of the general formula (I) 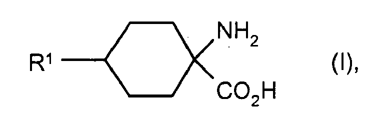 in which
R¹ represents OR³,
where
R³ represents alkyl.

10. Compounds of the formula (I) according to Claim 9,
in which
R¹ represents OR³,
where
R³ represents C₁-C₄-alkyl.

11. Compounds of the formula (I) according to Claim 9,
in which
R¹ represents OR³,
where
R³ represents methyl or ethyl.

12. Compounds of the formula (Ia) 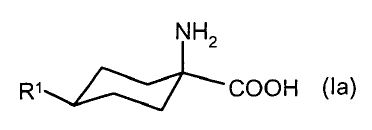 in which
R¹ is as defined above.

13. Compounds of the formula (Ib) 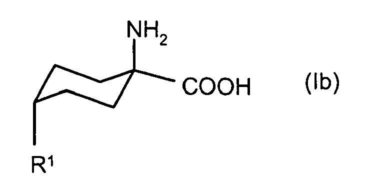 in which
R¹ is as defined above.

## Revendications

1. Composés de la formule (III) : 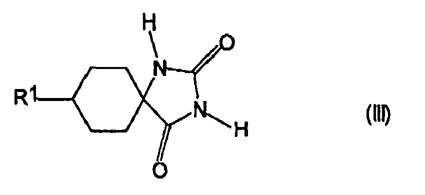 dans laquelle :
R¹ représente OR³ et R³ représente un radical alcoyle.

2. Composé de la formule (III) selon la revendication 1, dans lequel R³ représente un radical alcoyle en C₁-C₄.

3. Composé de la formule (III) selon la revendication 1, dans lequel R³ représente le radical méthyle, éthyle, n-propyle, n-butyle ou i-butyle.

4. Composés de la formule (III-a) selon la revendication 1 : 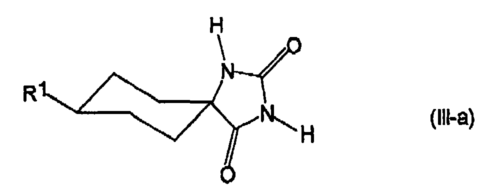 dans lesquels :
R¹ a la signification indiquée ci-dessus.

5. Composés de la formule (III-b) selon la revendication 1 : 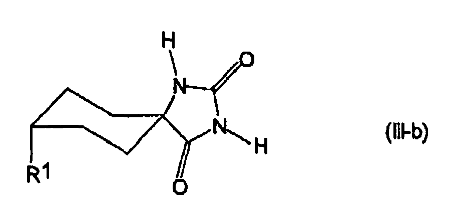 dans lesquels :
R¹ a la signification indiquée ci-dessus.

6. Procédé de préparation des composés de la formule (III) : 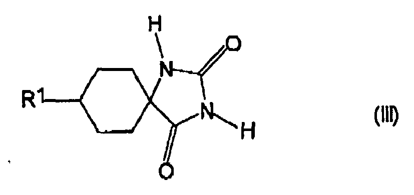 dans laquelle :
R¹ a la signification indiquée ci-dessus,
**caractérisé en ce que** l'on fait réagir les composés de la formule (II) : 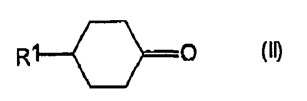 dans laquelle :
R¹ a la signification indiquée ci-dessus,
avec le carbonate d'ammonium et un cyanure d'alcoyle ou le cyanure de triméthylsilyle (TMSCN) dans l'eau comme solvant.

7. Procédé de préparation des composés de la formule (III-a) selon la revendication 6 : 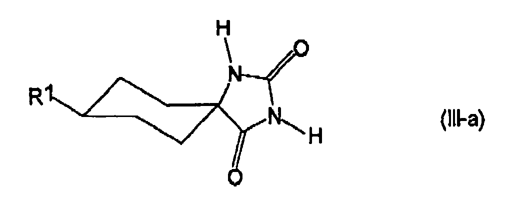 dans laquelle :
R¹ a la signification indiquée ci-dessus,
**caractérisé en ce que** l'on fait réagir les composés de la formule (II) : 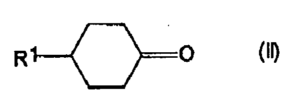 dans laquelle :
R¹ a la signification indiquée ci-dessus,
avec un cyanure d'alcoyle et le carbonate d'ammonium dans l'eau.

8. Procédé d'isolement des composés de la formule (III-a) : 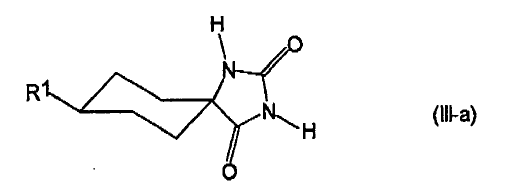 dans laquelle :
R¹ a la signification indiquée ci-dessus,
**caractérisé en ce que** l'on traite les composés de la formule (III) avec de l'ammoniaque aqueux.

9. Composés de la formule générale (I) : 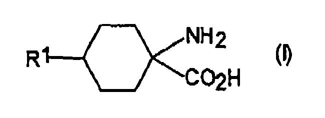 dans laquelle :
R¹ représente OR³,
où R³ représente un radical alcoyle.

10. Composés de la formule (I) selon la revendication 9,
dans lesquels :
R¹ représente OR³,
où R³ représente un radical alcoyle en C₁-C₄.

11. Composés de la formule (I) selon la revendication 9,
dans lesquels :
R¹ représente OR³,
où R³ représente le radical méthyle ou éthyle.

12. Composés de la formule (I-a) : 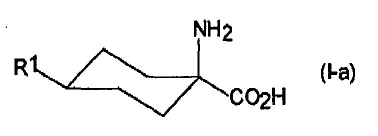 dans laquelle :
R¹ a la signification indiquée ci-dessus.

13. Composés de la formule (I-b) : 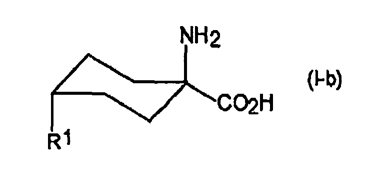 dans laquelle :
R¹ a la signification indiquée ci-dessus.
